# EUROPEAN PATENT SPECIFICATION

(11) **EP 3 329 855 B1**
(45) Date of publication and mention of the grant of the patent: **07.08.2019**
(21) Application number: 17171587.3
(22) Date of filing: 17.05.2017
(51) Int. Cl.: A61B 8/00, G01S 7/52

(54) **ULTRASONIC PROBE AND ULTRASONIC DIAGNOSTIC APPARATUS INCLUDING THE SAME**
ULTRASCHALLSONDE UND ULTRASCHALLDIAGNOSEVORRICHTUNG DAMIT
SONDE ULTRASONIQUE ET DISPOSITIF ET APPAREIL ULTRASONIQUE L'INCLUANT

(30) Priority: 02.12.2016 KR 20160163445
(43) Date of publication of application: 06.06.2018
(73) Proprietor: Samsung Medison Co., Ltd., Hongcheon-gun, Gangwon-do, 25108 (KR)
(72) Inventor: SONG, Chang Wook, Seoul (KR); JIN, Gil-Ju, Seoul (KR); KANG, Hak Il, Gyeonggi-do (KR); KIM, Chan Mo, Seoul (KR)
(74) Representative: Grootscholten, Johannes A.M.

(56) References cited:
- US-A1- 2003 013 959
- US-A1- 2003 114 754
- US-A1- 2009 043 203
- US-A1- 2016 049 066
- US-B1- 6 471 651

## Description

### BACKGROUND

### 1. Field

Embodiments of the present disclosure relate to an ultrasonic probe and an ultrasonic diagnostic apparatus, and more particularly, to technique of displaying basic information regarding operations of an ultrasonic probe and an ultrasonic diagnostic apparatus on a display with low power consumption so that a user may easily view information regarding the ultrasonic probe and the ultrasonic diagnostic apparatus at any time.

### 2. Description of the Related Art

An ultrasonic diagnostic apparatus is an apparatus which emits an ultrasonic signal toward a specific portion of an object, receives an ultrasonic signal (an ultrasonic echo signal) reflected from the object, and noninvasively obtains a tomographic image of soft tissue of the object or an image of a blood flow using information of the received ultrasonic signal.

The ultrasonic diagnostic apparatus has a small size and is cheap, compared to other image diagnostic apparatuses such as an X-ray diagnostic apparatus, an X-ray computerized tomography (CT) scanner, a magnetic resonance image (MRI) apparatus, a nuclear medicine diagnostic apparatus, etc.

Furthermore, the ultrasonic diagnostic apparatus is capable of obtaining an image regarding the inside of the object in real time, and is free from radiation exposure and thus very safe. Thus, in general, the ultrasonic diagnostic apparatus has been widely used in the fields of a cardiac diagnosis, an abdominal diagnosis, a urologic diagnosis, an obstetric and gynecologic diagnosis, etc.

Thus, the ultrasonic diagnostic apparatus includes an ultrasonic probe which transmits an ultrasonic signal to the object and receives an ultrasonic echo signal reflected from the object so as to obtain an ultrasonic image of the inside of the object.

The ultrasonic probe includes a piezoelectric layer which converts an electrical signal into a sound signal or converts a sound signal into an electrical signal as a piezoelectric material included in the ultrasonic probe vibrates, a matching layer which reduces the difference between sound impedances of the piezoelectric layer and the object to effectively transfer ultrasonic waves generated by the piezoelectric layer to the object, a lens which concentrates ultrasonic waves propagating toward the front of the piezoelectric layer on a specific point, a sound absorbing layer which blocks ultrasonic waves from propagating toward the rear of the piezoelectric layer so as to prevent distortion of an image, and the like.

In general, a display is likely to be included in an ultrasonic probe and an ultrasonic diagnostic apparatus. In the related art, the display simply displays a captured image or a user has to manipulate the display many times to view information required to control the ultrasonic probe and the ultrasonic diagnostic apparatus.

In particular, when the ultrasonic probe and the ultrasonic diagnostic apparatus are not used for a predetermined time or more and a screen of the display is thus automatically off, a user should press a specific button or turn on the screen again to view information regarding operations of the ultrasonic probe and the ultrasonic diagnostic apparatus.

Document US2009/043203A1 discloses an ultrasound probe with a touch screen.

Document US2016/049066A1 discloses an ultrasonic apparatus with operation state indicators.

Document US2003114754A1 discloses an ultrasonic apparatus with an OLED screen.

### SUMMARY

Therefore, it is an aspect of the present disclosure to direct displaying information regarding an ultrasonic probe and an ultrasonic diagnostic apparatus on displays of the ultrasonic probe and the ultrasonic diagnostic apparatus all the time, so that a user may easily view the information regarding the ultrasonic probe and the ultrasonic diagnostic apparatus at any time.

Additional aspects of the disclosure will be set forth in part in the description which follows and, in part, will be obvious from the description, or may be learned by practice of the disclosure.

In accordance with one aspect of the present disclosure, a ultrasonic probe comprises the features in assembly of the appended independent claim.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a perspective view of an ultrasonic diagnostic apparatus including an ultrasonic probe in accordance with an embodiment.
FIG. 2 is a block diagram illustrating elements of the ultrasonic diagnostic apparatus.
FIG. 3 illustrates the exteriors of various types of ultrasonic probes classified according to the shape of a transducer thereof.
FIG. 4 is a diagram illustrating various pictures which may be displayed on the first display 150 in accordance with an embodiment of the present disclosure.
FIG. 5(a) illustrates the first screen displayed on the first display in accordance with an embodiment of the present disclosure. FIG. 5(b) and (c) are diagrams comparing a principle of operating a panel of an LCD and a principle of operating a panel of an OLED with each other.
FIG. 6 is a cross-sectional view of a basic structure of an OLED.
FIG. 7 is a diagram illustrating a principle of operating an OLED.
FIG. 8 is a diagram illustrating various locations of a first display on an ultrasonic probe in accordance with an embodiment of the present disclosure.
FIG. 9 is a diagram illustrating the ultrasonic probe connected to various types of ultrasonic diagnostic apparatuses.
FIG. 10 illustrates a first picture including information identifying an ultrasonic diagnostic apparatus among information regarding an operating state of the ultrasonic probe.
FIG. 11 illustrates a first picture including moving-image identification information among information regarding an operating state of the ultrasonic probe.
FIG. 12 illustrates switching from a first picture to a third picture.
FIG. 13 illustrates a first picture including program identification information 163 among information regarding an operating state of the ultrasonic probe.
FIG. 14 illustrates a first picture including ultrasonic probe identification information among information regarding an operating state of the ultrasonic probe.
FIG. 15 illustrates a first picture including the information regarding the capacity of the battery.
FIG. 16 illustrates a first picture including the information representing whether the battery is in charge or not and a battery charging method and a charging message.
FIG. 17 illustrates a user interface picture through which a user may set types of information regarding an operating state of the ultrasonic probe to be included in a first picture.
FIG. 18 illustrates the exterior of an ultrasonic diagnostic apparatus in accordance with another embodiment of the present disclosure.
FIG. 19 illustrates various information as examples of the fourth picture in accordance with another embodiment of the present disclosure.
FIGS. 20 and 21 illustrate an ultrasonic diagnostic apparatus in accordance with another embodiment of the present disclosure.

### DETAILED DESCRIPTION

Embodiments set forth herein and elements illustrated in the drawings are merely examples of the present disclosure. There would have been variously modified examples which may replace the embodiments and the drawings at the filing date of the present application.

The terminology used herein is for the purpose of describing particular embodiments only and is not intended to be limiting of the present disclosure. As used herein, the singular forms 'a', 'an' and 'the' are intended to include the plural forms as well, unless the context clearly indicates otherwise.

It will be further understood that the terms 'comprise' and/or 'comprising,' when used in this specification, specify the presence of stated features, integers, steps, operations, elements, and/or components, but do not preclude the presence or addition of one or more other features, integers, steps, operations, elements, components, and/or groups thereof.

It will be understood that, although the terms first, second, third, etc., may be used herein to describe various elements, components, regions, layers and/or sections, these elements, components, regions, layers and/or sections should not be limited by these terms.

Hereinafter, embodiments of the present disclosure will be described in detail with reference to the accompanying drawings so that those of ordinary skill in the art can easily accomplish them.

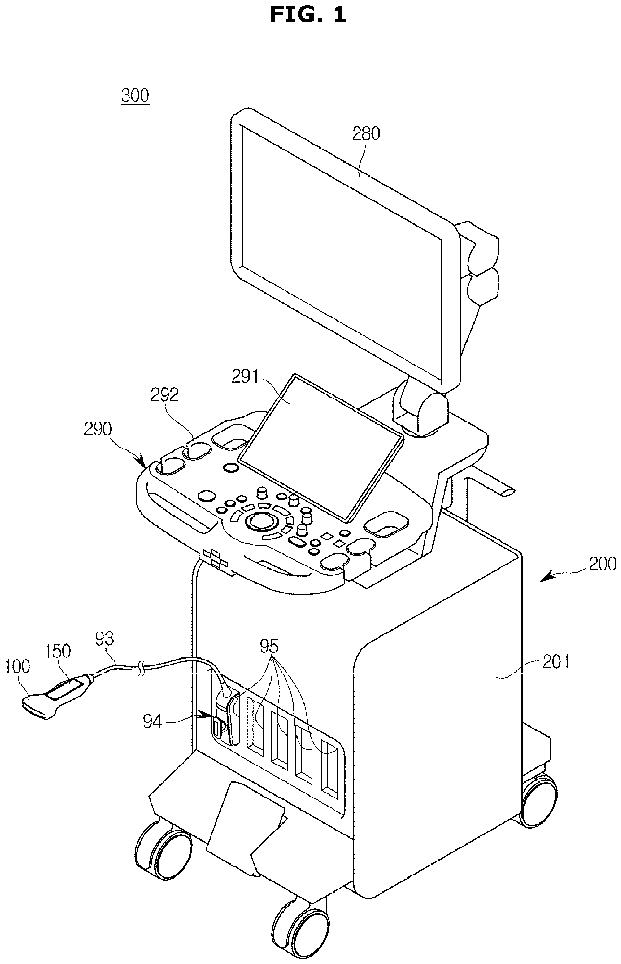

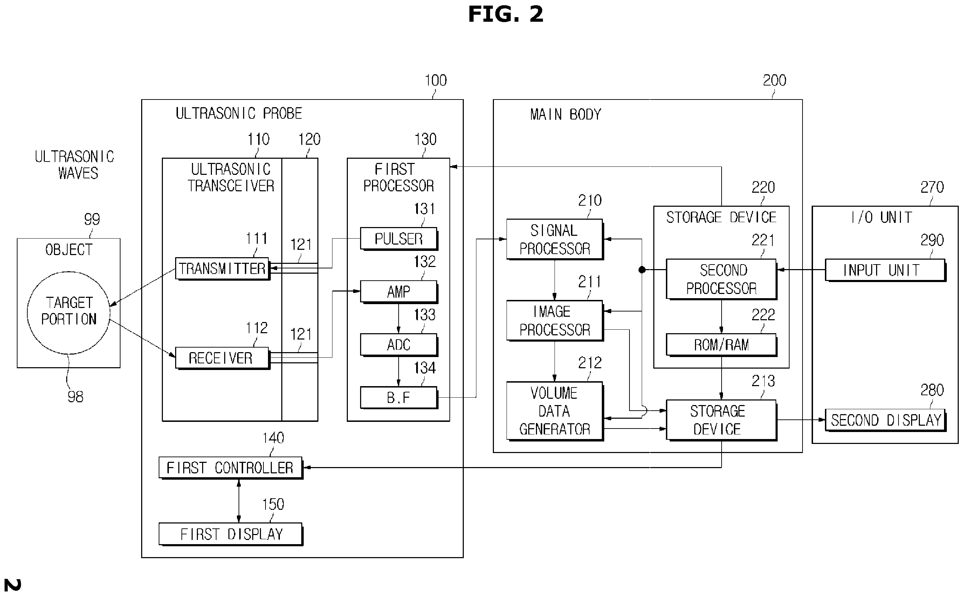

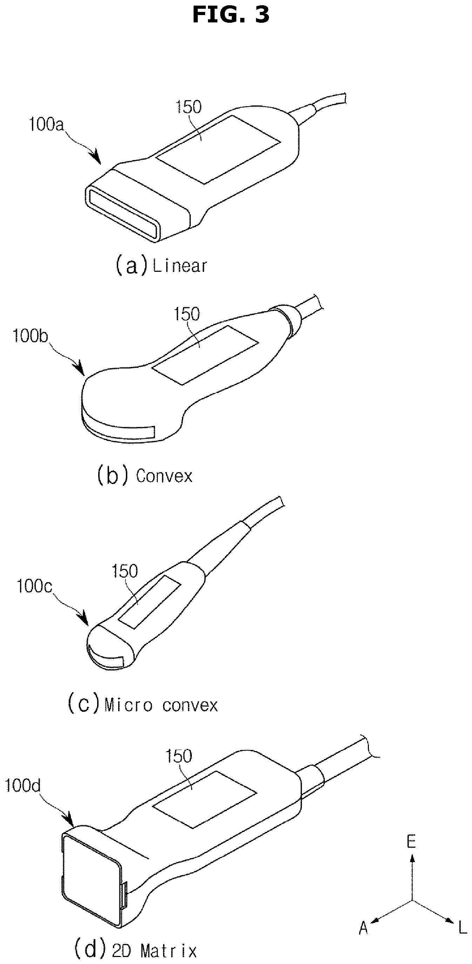

FIG. 1 is a perspective view of an ultrasonic diagnostic apparatus 300 including an ultrasonic probe 100 in accordance with an embodiment. FIG. 2 is a block diagram illustrating elements of the ultrasonic diagnostic apparatus 300. FIG. 3 illustrates the exteriors of various types of ultrasonic probes 100a to 100d classified according to the shape of a transducer thereof.

Referring to FIGS. 1 and 2, the ultrasonic diagnostic apparatus 300 may include a main body 200, an input unit 290 which receives a command for controlling the ultrasonic diagnostic apparatus 300 from a user, and a second display 280 which outputs information received from the main body 200.

In detail, the main body 200 may control overall operations of the ultrasonic diagnostic apparatus 300. Thus, various elements may be provided to control overall operations of either the ultrasonic probe 100 or the main body 200 of the ultrasonic diagnostic apparatus 300. The main body 200 and the ultrasonic probe 100 may transmit data to or receive data from each other using a connection cable 93 or a wireless communication module.

As illustrated in FIG. 1, the ultrasonic probe 100 and the main body 200 may be connected via the connection cable 93 to communicate with each other. An electrical signal output from the ultrasonic probe 100 may be transmitted to the main body 200 via the connection cable 93. Similarly, a control command generated by the main body 200 and the like may be transmitted to the ultrasonic probe 100 via the connection cable 93.

A connector 94 may be provided on one end of the connection cable 93. The connector 94 may be connected to or disconnected from a port 95 provided on an outer covering 201 of the main body 200. When the connector 94 is connected to the port 95, the ultrasonic probe 100 and the main body 200 may be connected to each other to communicate with each other.

A probe holder 292 may be provided on one side surface of the main body 200 to hold the ultrasonic probe 100 therewith. A number of probe holders 292 may correspond to the number of ultrasonic probes 100. The probe holder 292 may be attached to or detached from the main body 200. When the ultrasonic probe 100 is not in use, a user may store the ultrasonic probe 100 by holding the ultrasonic probe 100 with the probe holder 292.

Furthermore, the main body 200 may receive an electrical signal output from the ultrasonic probe 100 and transmit an electrical signal generated by the main body 200 to the ultrasonic probe 100 via a wireless communication network. In this case, a wireless communication module including an antenna and a wireless communication chip may be installed in each of the ultrasonic probe 100 and the main body 200.

The wireless communication module may be a short-range wireless communication module using at least one among Bluetooth, Bluetooth low energy, infrared data association (IrDA), Wi-Fi, Wi-Fi Direct, Ultra-Wideband (UWB), and Near-Field Communication (NFC), or may be a wireless communication module supporting a 3GPP-, 3GPP2-, or IEEE-based wireless communication network authenticated by the International Telecommunication Unit (ITU).

The main body 200 may exchange data with a server of a hospital or another medical device in the hospital connected to the main body 200 through a medical image information system (a picture archiving and communication system (PACS)) via a communication unit. The main body 200 may exchange data according to digital imaging and communications in medicine (DICOM) standards. However, embodiments are not limited thereto.

The second display 280 may be coupled to the main body 200 and output various information received from the main body 200.

In detail, the second display 280 may display an ultrasonic image of a target inner portion of an object. The ultrasonic image displayed on the second display 280 may be a two-dimensional (2D) ultrasonic image or a three-dimensional (3D) ultrasonic image. Various ultrasonic images may be displayed on the second display 280 according to an operating mode of the ultrasonic diagnostic apparatus 300.

According to an embodiment, examples of the ultrasonic image include an amplitude mode (A-mode) image, a brightness mode (B-Mode) image, a motion mode (M-mode) image, a color mode (C-mode) image, and a Doppler mode (D-mode) image.

As used herein, the A-mode image means an ultrasonic image representing the intensity of an ultrasonic signal corresponding to an echo ultrasonic signal, the B-mode image means an ultrasonic image representing the intensity of the ultrasonic signal corresponding to the echo ultrasonic signal using brightness, and the M-mode image means an ultrasonic image representing motion of an object at a specific location according to time. The D-mode image means an ultrasonic image representing a moving object in the form of waveforms according to the Doppler effect. The C-mode image means an ultrasonic image representing a moving object in the form of color spectrums.

Thus, the second display 280 may be embodied as well-known various displays, such as a cathode ray tube (CRT), a liquid crystal display (LCD), a light-emitting diode (LED), a plasma display panel (PDP), an organic light-emitting diode (OLED), etc.

The input unit 290 may be embodied variously, e.g., as a keyboard, a foot switch, or foot pedal, or the like.

For example, the keyboard may be embodied by hardware. In this case, the keyboard may include at least one among a switch, a key, a joystick, and a track ball. Alternatively, the keyboard may be embodied by software, e.g., as a graphical user interface (GUI). In this case, the keyboard may be displayed on the second display 280.

When the second display 280 is a touch screen type display, the second display 280 may also serve as the input unit 290. That is, the main body 200 may receive various commands from a user through at least one of the second display 280 and the input unit 290. In one embodiment, a third display 291 of FIG. 1 is capable of performing both a display function and an input function.

The second display 280 and the input unit 290 may be defined together as an input/output (I/O) unit 270 since they may receive information from a user or transmit information to the user.

The main body 200 of the ultrasonic diagnostic apparatus 300 has been described above with reference to FIG. 1. The ultrasonic probe 100 will be described with reference to FIGS. 2 and 3 below.

Referring to FIG. 2, the ultrasonic probe 100 may include an ultrasonic transceiver 110 configured to generate or receive ultrasonic waves, a first processor 130 which is electrically connected to the ultrasonic transceiver 110 and controls an operation of the ultrasonic transceiver 110 or performs signal processing using an electrical signal output from an ultrasonic element, a first controller 140 which transmits information received from the main body 200 of the ultrasonic diagnostic apparatus 300 or information set by a user to a first display 150, and the first display 150 which outputs the information received from the first controller 140.

The ultrasonic transceiver 110 may include an ultrasonic transducer which may generate ultrasonic waves or an electrical signal corresponding to the ultrasonic waves. The ultrasonic transducer may generate ultrasonic waves by converting alternating-current (AC) energy of a predetermined frequency into mechanical vibration of the predetermined frequency or convert mechanical vibration of a predetermined frequency based on received ultrasonic waves into AC energy. Thus, the ultrasonic transducer may generate ultrasonic waves or output an electrical signal corresponding to receive ultrasonic waves.

Referring to the embodiment of FIG. 2, the ultrasonic transceiver 110 may include an ultrasonic transmitter 111 and an ultrasonic receiver 112.

The ultrasonic transmitter 110a may generate ultrasonic waves of a frequency corresponding to a frequency of a pulse signal according to the pulse signal transmitted by the first processor 130 or a second processor 221. The generated ultrasonic waves may be emitted to a target portion 98 of an object 99.

The ultrasonic receiver 110b may receive ultrasonic waves reflected from the target portion 98 of the object 99 or generated from the target portion 98 by laser or the like, and convert a received signal into an ultrasonic signal. The ultrasonic receiver 110b may include a plurality of ultrasonic transducers. The ultrasonic transducers respectively output ultrasonic signals. Thus, the ultrasonic receiver 110b may output ultrasonic signals of a plurality of channels.

The ultrasonic transceiver 110 may be installed on a surface of a sound absorbing unit 120. The sound absorbing unit 120 may include a first connection unit 121 corresponding to the ultrasonic transceiver 110.

In one embodiment, the first connection unit 121 may be installed in the sound absorbing unit 120 to pass through the sound absorbing unit 120. In this case, the first connection unit 121 may penetrate from one surface of the sound absorbing unit 120 to another surface thereof.

The first processor 130 may generate and output an electrical signal for controlling the ultrasonic transceiver 110, or perform various signal processings using an ultrasonic signal transmitted from the ultrasonic transceiver 110.

The electrical signal output from the first processor 130 may be transmitted to the ultrasonic transceiver 110, for example, the ultrasonic transmitter 110a, via the first connection unit 121. The ultrasonic transmitter 110a may be driven by the electrical signal transmitted thereto.

According to the embodiment of FIG. 2, the first processor 130 may include at least one among a pulser 131, an amplifier (AMP) 132, an analog-to-digital converter (ADC) 133, and a beamformer (BF) 134.

The pulser 131 may generate a voltage of a predetermined frequency for driving the ultrasonic transceiver 110, and transmit the voltage to the ultrasonic transceiver 110. The ultrasonic transceiver 110 may vibrate according to the amplitude and frequency of the voltage output from the pulser 131 to generate ultrasonic waves.

The frequency and amplitude of the ultrasonic waves generated by ultrasonic transceiver 110 may be determined by the amplitude and frequency of the voltage generated by the pulser 131. The voltage output from the pulser 131 may be applied to the ultrasonic transceiver 110 after a predetermined time. Thus, the ultrasonic waves generated by the ultrasonic transceiver 110 may be concentrated on the target portion 98 or be steered in a predetermined direction.

In one embodiment, the pulser 131 may be included in the second processor 221. In this case, the first processor 130 may not include the pulser 131.

The AMP 132 may amplify an ultrasonic signal output from the ultrasonic receiver 110b of the ultrasonic transceiver 110. In one embodiment, the AMP 132 may differently amplify ultrasonic signals of a plurality of channels output from a plurality of ultrasonic transceivers 110, thereby compensating for the differences between the intensities of the ultrasonic signals of the plurality of channels.

When the amplified ultrasonic signal is an analog signal, the ADC 133 may convert the amplified ultrasonic signal into a digital signal. The ADC 133 may output a digital signal by sampling the ultrasonic signal which is an analog signal at a predetermined sampling ratio.

The BF 134 may concentrate ultrasonic signals input via a plurality of channels. The BF 134 may generate a beamformed signal by concentrating a signal transmitted from the ultrasonic transceiver 110, the AMP 132, or the ADC 133. The BF 134 may perform electronic-beam scanning, steering, concentrating, apodizing, and a calibration function on signals of a plurality of channels.

The first controller 140 may display various pictures on the first display 150 on the basis of information received from the main body 200, and transmit information received from a user via the first display 150 to the main body 200.

In detail, the first controller 140 may sense an operating state of the ultrasonic probe 100, and display a first picture 160, a second picture 180, a third picture 190, etc. including information regarding operating states of the ultrasonic probe 100, which are set beforehand by a user, on the first display 150 according to the sensed operating state. In some cases, the first controller 140 may control the brightness of the first to third pictures 160 to 190 and the number of colors to be expressed.

Furthermore, FIG. 2 illustrates one controller, i.e., the first controller 140, as a controller for controlling overall operations of the ultrasonic probe 100 but a controller for controlling the first display 150 when the ultrasonic probe 100 is in a standby state may be provided separately in one embodiment. That is, the first controller 140 which controls overall operations of the ultrasonic probe 100 and a controller which controls a picture displayed on the first display 150 may be provided independently.

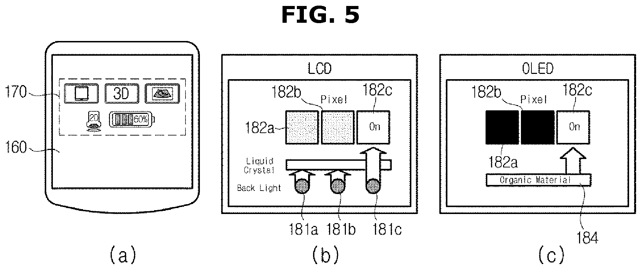

As described above, if an additional controller is provided, when the ultrasonic probe 100 enters the standby state, the first controller 140 which performs a relatively large number of operations may be powered off and only the additional controller which performs a relatively small number of operations may be driven, thereby increasing power efficiency. The controller which controls the first display 150 may be referred to variously as a controller of the first display 150, a standby-screen controller, a controller of an always-on-display (AOD) controller, or the like. The standby screen and the AOD controller will be described in detail with reference to FIG. 5 below.

The first display 150 may output information received from the first controller 140. When the first display 150 is embodied as a touch screen type display, it may receive various commands for manipulating the ultrasonic probe 100 from a user.

Thus, a panel of the first display 150 may be embodied as an LCD panel, an LED panel, an OLED panel, or the like. Any panel capable of outputting an image may be employed as the panel of the first display 150.

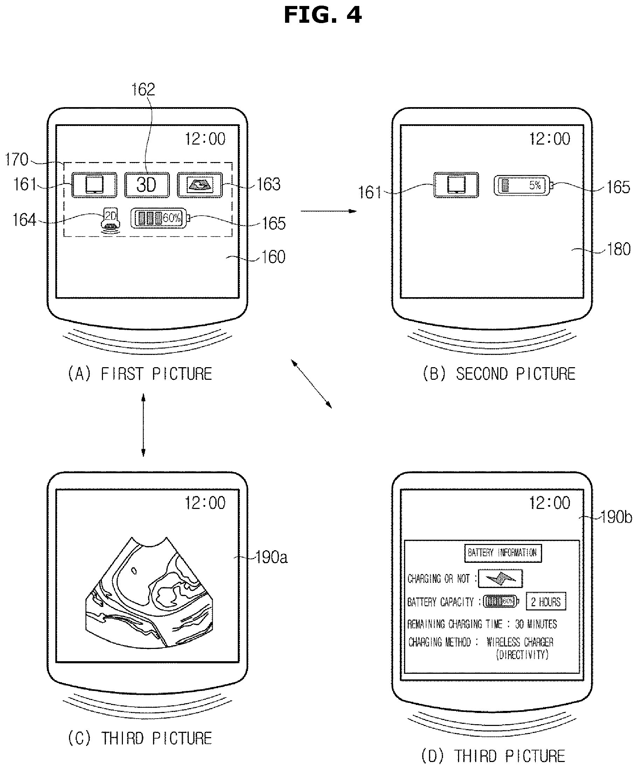

In accordance with an embodiment of the present disclosure, a plurality of pictures may be displayed on the first display 150 with low power consumption particularly when the panel of the first display 150 is embodied as an OLED panel, as will be described in more detail with reference to FIGS. 4 and 5 below.

When the ultrasonic probe 100 is a wireless ultrasonic probe, a battery (not shown) which supplies power to the ultrasonic probe 100 may be further provided. In this case, the first controller 140 may control the types and colors of information to be included on the first picture 160 on the basis of the capacity of the battery, as will be described in detail with reference to FIG. 4 below.

FIG. 3 illustrates the exteriors of various types of ultrasonic probes 100 classified according to a shape of the ultrasonic transceiver 110.

An ultrasonic probe 100a illustrated in FIG. 3(a) is a linear probe in which transducers are arranged in a straight line.

An ultrasonic probe 100b illustrated in FIG. 3(b) is a convex ultrasonic probe which has a convex surface and thus through which a fan-shape image is generated. The ultrasonic probe 100b is mainly used to check a large area, such as the abdomen. A basic principle of operating the ultrasonic probe 100b is the same as that of operating the ultrasonic probe 100a which is a linear probe.

An ultrasonic probe 100c illustrated in FIG. 3(c) is a micro-convex ultrasonic probe having the effect of a convex ultrasonic probe and designed to have a small size to easily inspect a narrow part of an object.

An ultrasonic probe 100d illustrated in FIG. 3(d) is a 2D matrix-array ultrasonic probe capable of providing a 3D ultrasonic diagnostic image which provides a 360° 3D image of an object in real time.

However, embodiments are not limited thereto, and the ultrasonic probe 100 may be another type of a probe well known in this art, such as a phased array probe or a 3D matrix probe, other than those illustrated in FIG. 3.

Three directions perpendicular to the ultrasonic probe 100, i.e., an axial direction A, a lateral direction L, and an elevation direction E, may be defined. A direction in which an ultrasonic signal is emitted may be defined as the axial direction A. A direction in which transducers are arranged in a row may be defined as the lateral direction L. A direction perpendicular to the axial direction A and the lateral direction L may be defined as the elevation direction E.

The outer and inner elements of the ultrasonic probe 100 and the ultrasonic diagnostic apparatus 300 have been described above. Features of the present disclosure will now be described.

An ultrasonic probe includes a display to inform a user of an operating state thereof. However, in the related art, generally, a captured image is simply displayed, and a user has to manipulate the display several times to determine an operating state of an ultrasonic probe even when the operating state of the ultrasonic probe is displayed.

In particular, when the ultrasonic probe is not used for a predetermined time or more, a screen of the display is automatically 'off' to prevent waste of power. In this case, a user may be inconvenienced since the user has to press a particular button or turn on the screen again to check the operating state of the ultrasonic probe.

In contrast, in the ultrasonic probe 100 and the ultrasonic diagnostic apparatus 300 in accordance with an embodiment of the present disclosure, information regarding operating states of the ultrasonic probe 100 and the ultrasonic diagnostic apparatus 300 may be provided to a user at any time through the first display 150 of the ultrasonic probe 100 and the second display 280 of the ultrasonic diagnostic apparatus 300. Thus, the user may easily notice the operating states of the ultrasonic probe 100 and the ultrasonic diagnostic apparatus 300, as will be described in detail with reference to FIG. 4 below.

FIG. 4 is a diagram illustrating various pictures which may be displayed on the first display 150 in accordance with an embodiment of the present disclosure. FIG. 4(a) and (b) illustrate a first picture 160 and a second picture 180 which may be displayed on the first display 150 when the ultrasonic probe 100 is in the standby state. FIG. 4(c) and (d) illustrate third pictures 190a and 190b which may be displayed on the first display 150 when a user manipulates the first display 150 in the standby state of the ultrasonic probe 100.

In detail, the standby state means a state in which the ultrasonic probe 100 is not in use by the user any longer, and should be understood to include a case in which an input is not received from the user for a first time, a case in which the user presses a lock button of the ultrasonic probe 100, and a case in which the ultrasonic probe 100 is mounted in the probe holder 292 of the ultrasonic diagnostic apparatus 300. In these cases, it may be determined that the ultrasonic probe 100 is not in use any longer and thus the first controller 140 is switched to a standby mode to prevent waste of power.

The first time means a time period in which manipulation of the ultrasonic probe 100 by the user is ended and it is highly probable that the ultrasonic probe 100 will not be in use any longer. For example, the first time may be one minute, two minutes, three minutes, or the like. However, the first time is not limited thereto, and may be variously changed according to an operating environment of the ultrasonic probe 100. The first time may be directly set by a user.

Thus, when the ultrasonic probe 100 is switched to the standby state, the first controller 140 may terminate a picture displayed on the first display 150 and display the first picture 160 on the first display 150 as illustrated in FIG. 4 (a) in order to prevent waste of power.

In detail, the first picture 160 is a picture which includes information regarding an operating state of the ultrasonic probe 100 and which is displayed on the first display 150 so that a user may view the information regarding the operating state of the ultrasonic probe 100 at any time without manipulating the ultrasonic probe 100.

The information regarding the operating state of the ultrasonic probe 100 may include various current information regarding the ultrasonic probe 100, e.g., information identifying a mode in which an image captured by the ultrasonic probe 100 is output and a display device from which the captured image is output. The information regarding the operating state of the ultrasonic probe 100 may further include information identifying an ultrasonic diagnostic apparatus being currently connected to the ultrasonic probe 100, information regarding whether a battery of the ultrasonic probe 100 is in charge or not, a charging method, etc. Types of information to be expressed may be set by a user.

Furthermore, the first picture 160 displayed in the standby state may be interchangeably referred to as an "AOD" picture. The AOD picture literally means a default picture, including specific information, displayed on a display in the standby state. A user may easily view predetermined information regarding an operating state of the ultrasonic probe 100 through the AOD picture. Thus, the AOD picture may have the same characteristics as those of the first picture 160 and characteristics of the first picture 160 which are to be described below may also apply to the AOD picture.

In addition, the information described above may be expressed in the form of a set of icons 170 as illustrated in FIG. 4 (a). In this case, a user may be able to intuitively know an operating state of the ultrasonic probe 100 through the set of icons 170.

FIG. 4(b) is a diagram illustrating an embodiment of the second picture 180 in which the type and brightness of information to be included in the second picture 180 are controlled according to a voltage state of a battery.

In detail, the first controller 140 may display the second picture 180 in which the type and brightness of information included in the first picture 160 are controlled on the basis of the capacity of the battery, after the ultrasonic probe 100 is switched to the standby state.

If the capacity of the battery is low, displaying of all information regarding the ultrasonic probe 100 is not preferable in terms of power efficiency. Thus, in this case, the first controller 140 may display only some information 161 and 165 similar to the second picture 180 of FIG. 4 (b), thereby reducing battery consumption. At the same time, the number of colors to be expressed in the second picture 180 may be decreased or brightness of the second picture 180 may be adjusted to increase battery efficiency. Similarly, the types of information to be included in the second picture 180 may be set by a user.

The second picture 180 of FIG. 4 (b) is a picture which may be displayed a predetermined time (a second time) after the ultrasonic probe 100 is switched to the standby state.

If a user does not manipulate the ultrasonic probe 100 for a long time even after the first time elapses and the ultrasonic probe 100 is switched to the standby state, continuation of displaying an operating state of the ultrasonic probe 100 is not preferable in terms of power efficiency. Thus, in this case, the first controller 140 may display only a minimum amount of information as illustrated in FIG. 4 (b) so that the second picture 180 may be displayed for a longer time.

Thus, the second time means a time period in which a user does not manipulate the ultrasonic probe 100 for a long time. For example, the second time may be six hours, twelve hours, twenty-four hours, or the like. However, the second time is not limited thereto and may be variously set according to an operating environment of the ultrasonic probe 100. The second time may be set by a user.

FIG. 4(c) and (d) are diagrams illustrating the third pictures 190a and 190b displayed on the first display 150.

The third pictures 190a and 190b mean pictures generally displayed when a user manipulates the ultrasonic probe 100 but are not limited thereto. The third pictures 190a and 190b should be understood to include, when a user touches a specific icon included in the first picture 160, a picture including information related to the touched icon. This is because the touching of the icon by the user may be also included in manipulation of the ultrasonic probe 100 by the user.

For example, FIG. 4(c) illustrates the third picture 190a in which, when a user touches information regarding an ultrasonic image included in the first picture 160, a program related to the ultrasonic image is run. FIG. 4(d) illustrates the third picture 190b, including information regarding a battery, displayed when a user touches battery information included in the first picture 160.

Accordingly, the first picture 160 of FIG. 4(a) may be switched to the third picture 190a or 190b of FIG. 4(c) or (d) through a user's manipulation as indicated by a direction of arrows of FIG. 4, and the third picture 190a or 190b may be switched to the first picture 160 when the user's manipulation is not performed for the first time.

In accordance with an embodiment of the present disclosure, an OLED panel may be mounted as the panel of the first display 150. In this case, the first picture 160 and the second picture 180 of FIG. 4 may be displayed with low power consumption, as will be described in detail with reference to FIGS. 5 to 7 below.

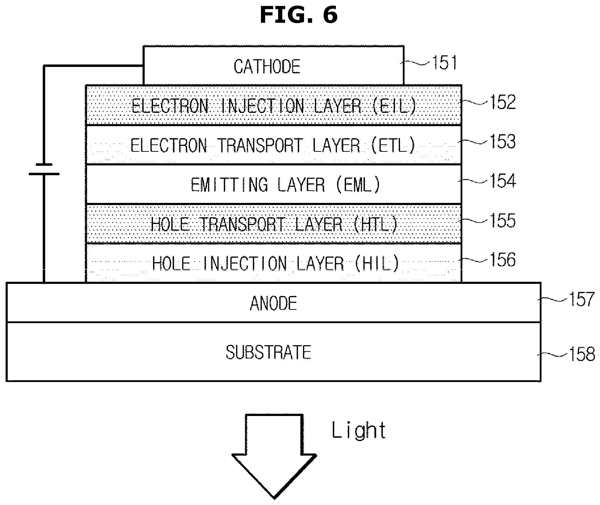

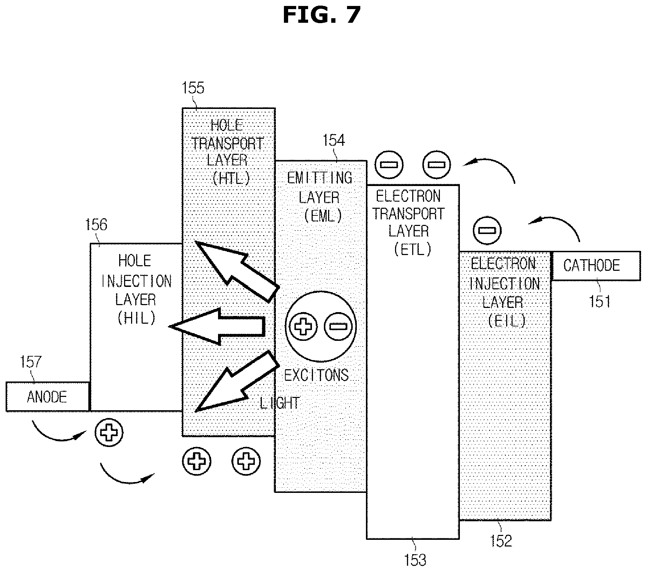

FIG. 5(a) illustrates the first screen 160 displayed on the first display 150 in accordance with an embodiment of the present disclosure. FIG. 5(b) and (c) are diagrams comparing a principle of operating a panel of an LCD and a principle of operating a panel of an OLED with each other. FIG. 6 is a cross-sectional view of a basic structure of an OLED. FIG. 7 is a diagram illustrating a principle of operating an OLED.

When the ultrasonic probe 100 enters the standby mode and thus the first controller 140 displays the first picture 160 on the first display 150, information may be displayed in only a region of the first picture 160, i.e., a region of the set of icons 170, and no information may be displayed in the remaining region of the first picture 160, as illustrated in FIG. 5(a).

In this case, it is preferable that elements of a display panel be not driven in relation to the remaining region displaying no information in terms of power efficiency. However, when an LCD is applied to the display panel, elements of the display panel are driven even in relation to the remaining region displaying no information according to characteristics of the LCD, thereby causing waste of power.

However, when an OLED panel is applied as the display panel, elements of the display panel may be driven only in relation to the region including the set of icons 170 and thus power efficiency may be increased. In particular, according to the present disclosure, power efficiency may be significantly increased in the case of a picture for which only a particular portion of the display panel may be driven.

In the LCD of FIG. 5(b) and 5(c), a backlight unit 181 is provided below a liquid crystal layer 184, and light emitted from the backlight unit 181 sequentially passes through the liquid crystal layer 184 and a color filter (not shown) and then emits unique light.

Pixels 182a and 182b are displayed in black, since although light is emitted thereto from backlight units 181a and 181b, the light cannot pass through the crystal layer 184 and thus no light passes through the color filter. Accordingly, according to the structural characteristics of the LCD, the backlight units 181a and 181b should be also driven for pixels which are to be displayed in black and thus power efficiency is low.

In contrast, in the case of the panel of the OLED, an organic light-emitting device 184 is a self-emitting device and thus each pixel emits light to express a color. Thus, as illustrated in FIG. 5(c), only a pixel 182c corresponding to a color to be expressed may be driven without driving the pixels 182a and 182b to be displayed in black, thereby significantly deceasing power consumption.

A structure and operating principle of the OLED will now be described in detail. As illustrated in FIG. 6, an OLED panel has a structure in which a transparent electrode layer 157 forming an anode (+) is coupled to a substrate 158 which is transparent, e.g., a glass substrate, multilayer organic thin films 152 to 156 having different transport capabilities are sequentially formed on the transparent electrode layer 157, and then another transparent electrode layer 151 forming a cathode (-) is coupled to a resultant structure. An indium tin oxide (ITO), an indium zinc oxide (IZO), or the like having a high work function may be used as the anode 157. A metal such as aluminum (Al), indium (In), magnesium (Mg), or calcium (Ca), an alloy thereof, or the like having a low work function may be used as the cathode 151.

The multilayer organic thin films 152 to 156 may include a hole injection layer (HIL) 156 for injecting holes from the anode 157, a hole transport layer (HTL) 155 for transporting the holes, an emitting layer (EML) 154 for generating light, an electron transport layer 153 for transporting electrons, and an electron injection layer (EIL) 152 for injecting electrons. A whole thickness of the multilayer organic thin films 152 to 156 may be about 100 nm. A material having a highest occupied molecular orbital (HOMO), for example, PEDOT such as PSS or Cu-PC, may be used as the HIL 156 to appropriately inject holes. A material having a lowest unoccupied molecular orbital (LUMO), for example, LiF, LiO, or CsF of about 0.5 to 1 nanometers, may be used as the EIL 152 to appropriately inject electrons. For a simple structure of the OLED, the EIL 152 of FIG. 2 may be omitted and the HIL 156 and the HTL 155 may be integrally formed with each other.

In the structure illustrated in FIG. 6, when a voltage is applied to the anode 157 and the cathode 151 to operate a display of the OLED, holes may be injected from the anode 157 and electrons may be injected from the cathode 151. The holes and the electrons may reach the EML 154 respectively via the HTL 155 and the ETL 153 as illustrated in FIG. 7. The electrons and the holes meeting one another at the EML 154 may be combined together to form excitons which are in an excited state. Energy emitted as the excitons change to a ground state is changed into light and then the light is emitted. The light generated in this case is emitted toward the anode 157. A wavelength of the emitted light is determined by the energy of the excitons, i.e., the difference between energies of the electrons and the holes.

When the panel of the first display 150 is embodied as a panel of an OLED, the OLED may be variously classified according to the type of a luminescent material, a light-emitting method, a light-emitting structure, a driving method, or the like. The OLED may be classified as a fluorescent type or a phosphorescent type according to the light-emitting method, and classified as a top emission structure or a bottom emission structure according to the light-emitting structure. Furthermore, the OLED may be classified as a passive matrix OLED or an active matrix OLED according to the driving method.

Thus, when the OLED panel described above is applied to the panel of the first display 150 in accordance with an embodiment of the present disclosure, the first picture 160 and the second picture 180, most regions of which are expressed in black may be displayed for a long time with low power consumption.

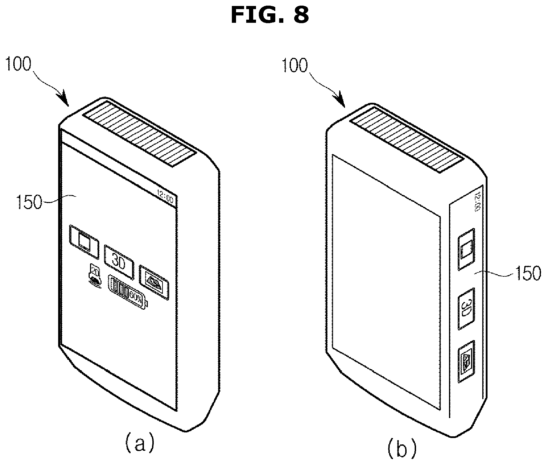

FIG. 8 is a diagram illustrating various locations of a first display 150 on an ultrasonic probe 100 in accordance with an embodiment of the present disclosure.

Referring to FIG. 8, a transducer is disposed on one side surface of the ultrasonic probe 100 and thus the first display 150 may be disposed on one side surface of a main body of the ultrasonic probe 100. Thus, the first display 150 may be disposed on a front portion of the main body of the ultrasonic probe 100 as illustrated in FIG. 8(a), or may be disposed on a side surface of the main body of the ultrasonic probe 100 as illustrated in FIG. 8(b). However, the location of the first display 150 is not limited thereto, and the first display 150 may be disposed on various locations, e.g., a bottom part of the main body of the ultrasonic probe 100, although not shown in FIG. 8.

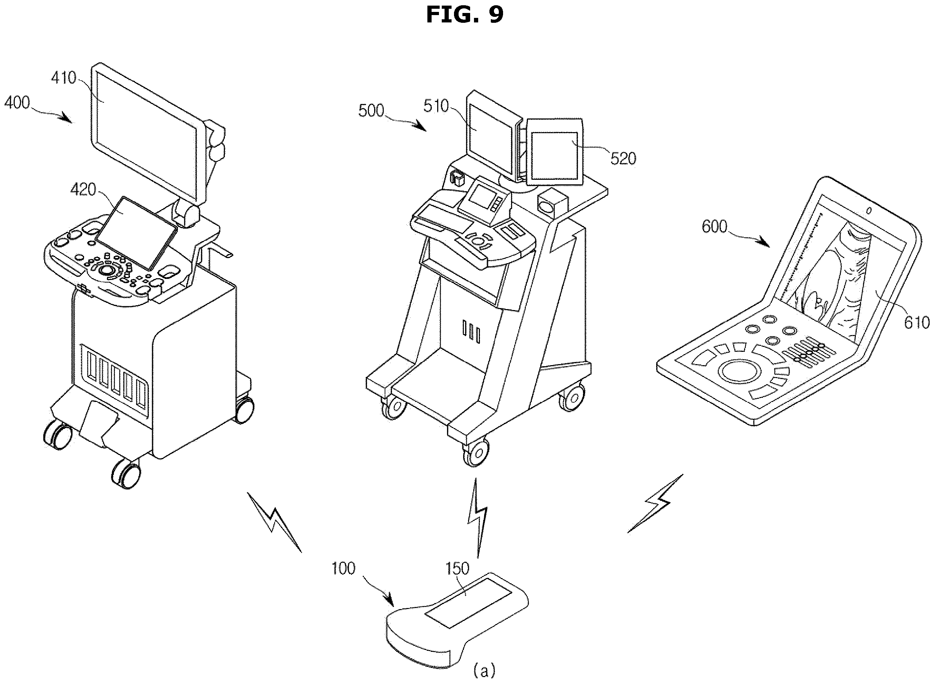

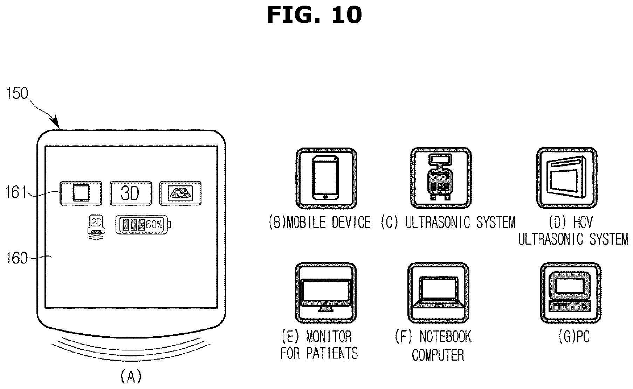

FIG. 9 is a diagram illustrating the ultrasonic probe 100 connected to various types of ultrasonic diagnostic apparatuses. FIG. 10(a) illustrates a first picture 160 including information 161 identifying an ultrasonic diagnostic apparatus among information regarding an operating state of the ultrasonic probe 100. FIG. 10(b) to (g) illustrate various information which may be included in the information 161 identifying an ultrasonic diagnostic apparatus.

As types of ultrasonic diagnostic apparatuses have been increased, use of one ultrasonic probe 100 by connecting it commonly to different types of ultrasonic diagnostic apparatuses 400, 500, and 600 has increased for simple equipment as illustrated in FIG. 9.

However, an ultrasonic probe according to the related art does not provide a user with information identifying an ultrasonic diagnostic apparatus being currently connected thereto and thus the user would feel inconvenience since the user should manipulate the ultrasonic probe to obtain this information.

In contrast, the ultrasonic probe 100 in accordance with an embodiment of the present disclosure is capable of providing a user with the information 161 identifying a device being currently connected to the ultrasonic probe 100 as illustrated in FIG. 10(a) so that the user may easily notice the device being currently connected to the ultrasonic probe 100.

In detail, the information 161 may be displayed as illustrated in FIG. 10(b) when a device being currently connected to the ultrasonic probe 100 is a mobile device, and displayed as illustrated in FIG. 10(c) when the device being currently connected to the ultrasonic probe 100 is an ultrasonic system. The information 161 may be displayed as illustrated in FIG. 10(d) when the device being currently connected to the ultrasonic probe 100 is an HCV ultrasonic system.

Furthermore, several display devices 410 and 420 may be provided in one ultrasonic diagnostic apparatus 400 as illustrated in FIG. 9. The ultrasonic probe 100 in accordance with an embodiment of the present disclosure may provide a user with not only information identifying an ultrasonic diagnostic apparatus being currently connected to the ultrasonic probe 100 but also information identifying a display device to which a currently captured image is output.

In detail, such identification information may be provided to a user as illustrated in FIG. 10(e) when the currently captured image is output to a monitor for patients, may be provided to the user as illustrated in FIG. 10(f) when the currently captured image is output to a notebook computer, and may be provided to the user as illustrated in FIG. 10(g) when the currently captured image is output to a personal computer (PC).

The information illustrated in FIGS. 10(b) to (f) is merely examples and embodiments are not limited thereto. Thus, the identification information may also include information identifying various devices which may be connected to the ultrasonic probe 100.

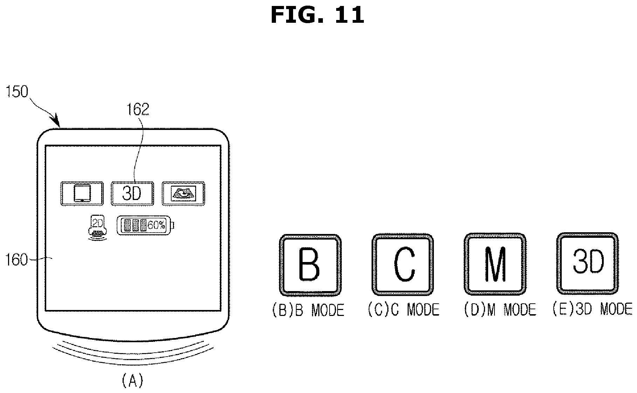

FIG. 11(a) illustrates a first picture 160 including moving-image identification information 162 among information regarding an operating state of the ultrasonic probe 100. FIG. 11(b) to (e) illustrate various information as examples of the moving-image identification information 162.

An ultrasonic image captured by the ultrasonic probe 100 may be variously output in the form of an A-mode image, a B-mode image, a C-mode image, an M-mode image, a D-mode image, a 3D-mode image, or the like according to a user's manipulation.

The A-mode image means an ultrasonic image representing the intensity of an ultrasonic signal corresponding to an echo ultrasonic signal. The B-mode image means an ultrasonic image representing, in the form of brightness, the intensity of an ultrasonic signal corresponding to an echo ultrasonic signal. The C-mode image means an ultrasonic image representing a moving object in the form of a color spectrum. The D-mode image means an ultrasonic image representing a moving object in the form of a waveform according to the Doppler effect. The M-mode image means an ultrasonic image representing movement of an object on a specific location according to time. The 3D-mode image represents a captured image in the form of a 3D image.

Thus, the moving-image identification information 162 may be displayed on the first picture 160 as illustrated in FIG. 10(b) when a moving image is a B-mode image, may be displayed on the first picture 160 as illustrated in FIG. 10(c) when the moving image is a C-mode image, may be displayed on the first picture 160 as illustrated in FIG. 10(d) when the moving image is a M-mode image, and may be displayed on the first picture 160 as illustrated in FIG. 10(e) when the moving information is a 3D-mode image, so that a user may notice the type of the moving image.

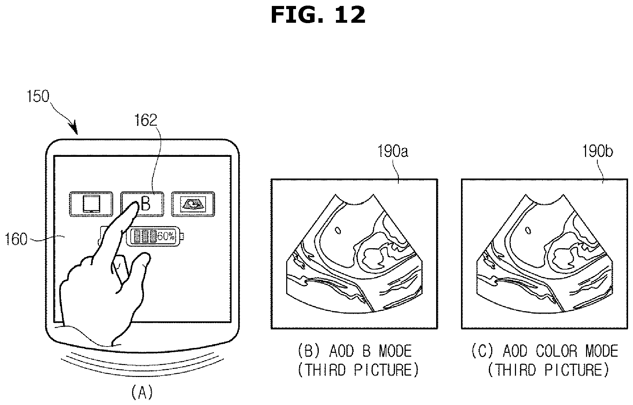

FIG. 12 illustrates switching from a first picture 160 to a third picture 190. FIG. 12(a) illustrates clicking moving-image identification information 162 on the first picture 160, performed by a user. FIG. 12(b) and (c) illustrate switching from the first picture 160 displayed on the first display 150 to the third picture 190 as the user clicks the moving-image identification information 162.

When the ultrasonic probe 100 is in a standby-mode state, the user may click information included in the first picture 160 to view information related to the clicked information or execute a program related to the clicked information.

In detail, as illustrated in FIG. 12(a), when the user touches the moving-image identification information 162, the third picture 190 may be output in the form of a B-mode image as illustrated in FIG. 12(b) and (c).

In this case, a color mode of the output third picture 190 may be expressed only in black and white as illustrated in FIG. 12(b) or may be expressed in various colors as illustrated in FIG. 12(c) according to a user's settings. The types of colors to be expressed may be also set by the user. In FIG. 12(c), different patterns represent different colors.

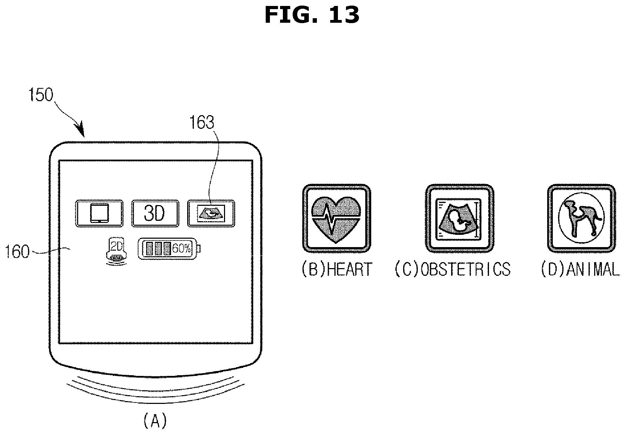

FIG. 13(a) illustrates a first picture 160 including program identification information 163 among information regarding an operating state of the ultrasonic probe 100. FIG. 13(b) to (d) illustrate various information as examples of the program identification information 163.

Since the ultrasonic probe 100 may use various programs, the first picture 160 may provide the program identification information 163 regarding a program which is currently being used in the ultrasonic probe 100 to avoid a user's confusion.

In detail, the program identification information 163 may be displayed as illustrated in FIG. 13(b) when the currently used program is a program for the heart, may be displayed as illustrated in FIG. 13(c) when the currently used program is related to obstetrics, and may be displayed as illustrated in FIG. 13(d) when the currently used program is related to animals so as to provide identification information to the user.

The information illustrated in FIG. 13(b) to (d) is merely examples and embodiments are not limited thereto. Thus, the program identification information 163 may be variously displayed for a program being currently used by the ultrasonic probe 100.

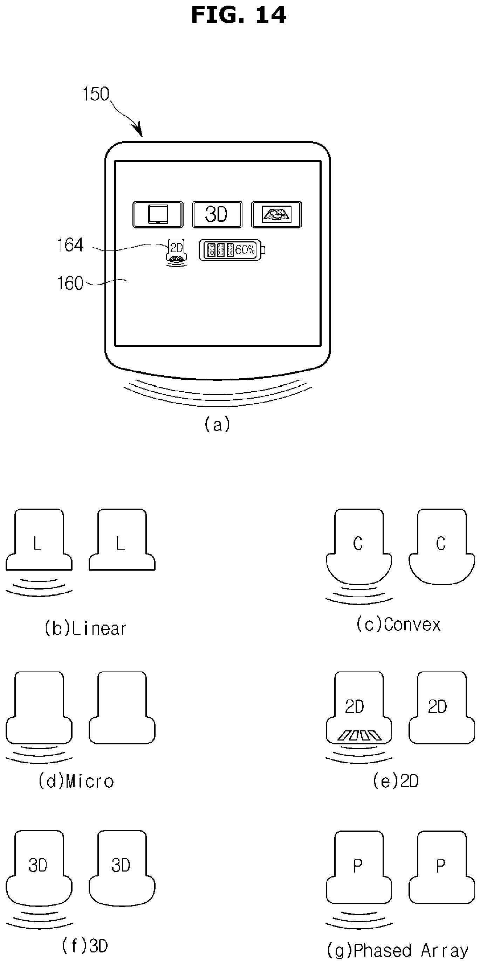

FIG. 14(a) illustrates a first picture 160 including ultrasonic probe identification information 164 among information regarding an operating state of the ultrasonic probe 100. FIG. 14(b) to (g) illustrate various information as examples of the ultrasonic probe identification information 164.

There are various types of ultrasonic probes as illustrated in FIG. 3 but a user would feel difficulties determining a type of an ultrasonic probe according to the shape thereof.

However, as illustrated in FIG. 14(a), when the ultrasonic probe identification information 164 is included in the first picture 160, a user may be able to easily recognize the type of the ultrasonic probe through the ultrasonic probe identification information 164.

In detail, the ultrasonic probe identification information 164 may be displayed as illustrated in FIG. 14(b) when an ultrasonic probe displaying the first picture 160 is the linear ultrasonic probe 100a, may be displayed as illustrated in FIG. 14(c) when the ultrasonic probe displaying the first picture 160 is the convex ultrasonic probe 100b, and may be displayed as illustrated in FIG. 14(d) when the ultrasonic probe displaying the first picture 160 is the micro-convex ultrasonic probe 100c. Furthermore, the ultrasonic probe identification information 164 may be displayed as illustrated in FIG. 14(e) when the ultrasonic probe displaying the first picture 160 is the 2D matrix-array ultrasonic probe 100d, may be displayed as illustrated in FIG. 14(f) when the ultrasonic probe displaying the first picture 160 is a 3D matrix-array ultrasonic probe (not shown), and may be displayed as illustrated in FIG. 14(g) when the ultrasonic probe displaying the first picture 160 is a phased array ultrasonic probe (not shown).

The ultrasonic probe identification information 164 may include information representing whether the ultrasonic probe 100 is wirelessly connected to an ultrasonic diagnostic apparatus at present, as well as information simply identifying the type of the ultrasonic probe 100.

In detail, the ultrasonic probe identification information 164 may be displayed as illustrated in a left diagram of FIG. 14(b) when the ultrasonic probe 100 is currently connected to the ultrasonic diagnostic apparatus, and may be displayed as illustrated in a right diagram of FIG. 14(b) when the ultrasonic probe 100 is not connected to the ultrasonic diagnostic apparatus, so that information regarding a connected state of the ultrasonic probe 100 may be provided intuitively to a user.

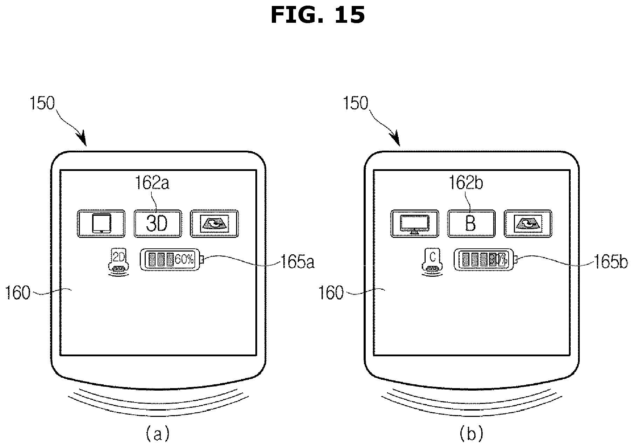

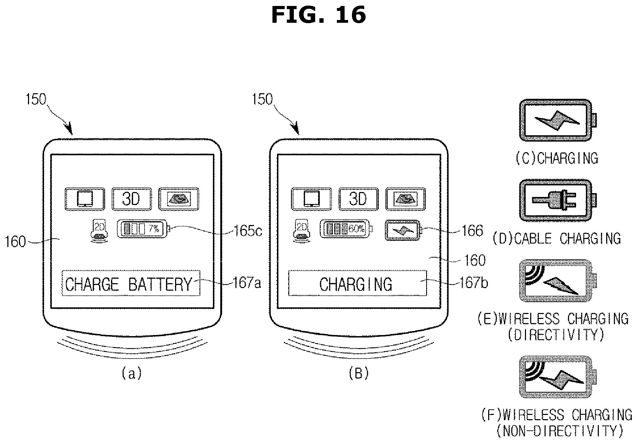

FIGS. 15 and 16 are diagrams illustrating a first picture 160 including information 165 and/or 166 identifying a battery among information regarding an operating state of the ultrasonic probe 100. FIG. 15 illustrates a first picture 160 including the information 165 regarding the capacity of the battery. FIG. 16 illustrates a first picture 160 including the information 166 representing whether the battery is in charge or not and a battery charging method and a charging message 167.

Referring to FIG. 15, the first picture 160 may include the information 165 regarding the capacity of the battery to inform a user of a current state of the battery. As illustrated in FIG. 15, a displayed number and a degree to which the inside of a battery icon is filled are differently displayed according to a charging capacity of the battery so that a user may more intuitively notice the current state of the battery.

When the capacity of a battery of the ultrasonic probe 100 is low, a charging message 167a may be included in the first picture 160 so that a user may intuitively notice that charging is needed as illustrated in FIG. 16 (a). When the battery of the ultrasonic probe 100 is in charge, charging icon information 166 or a message 167b may be included in the first picture 160 as illustrated in FIG. 16(b).

Furthermore, information regarding the battery charging method may be included in the first picture 160. This information may be displayed as illustrated in FIG. 16(d) when the battery is charged through a cable, may be displayed as illustrated in FIG. 16(e) when the battery is charged through directive wireless charging, and may be displayed as illustrated in FIG. 16(f) when the battery is charged through non-directive wireless charging.

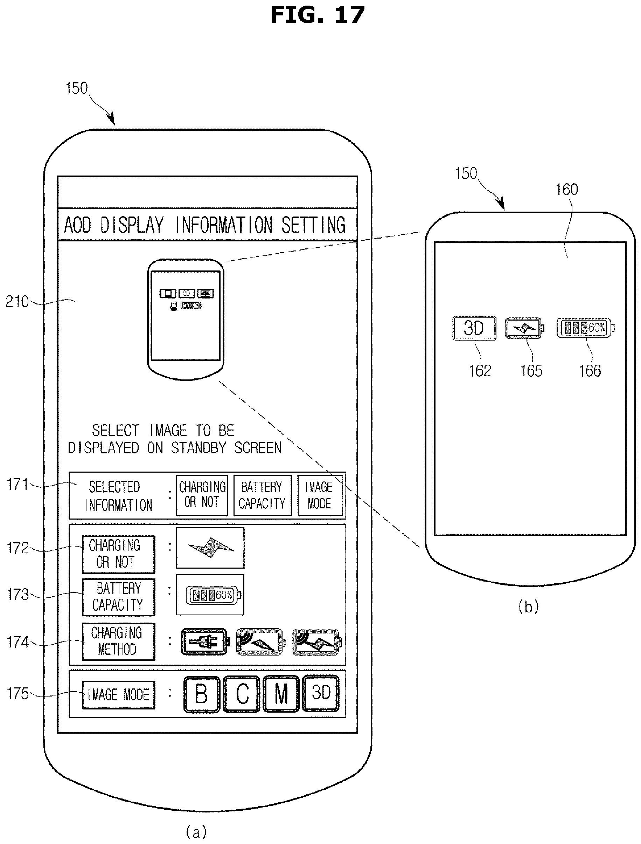

FIG. 17 illustrates a user interface (Ul) picture 210 through which a user may set types of information regarding an operating state of the ultrasonic probe 100 to be included in a first picture 160.

As illustrated in FIG. 17, a user may directly set types of information regarding the ultrasonic probe 100 to be included in the first picture 160 through the Ul picture 210. Thus, the user may be provided with information matching his or her preference.

In detail, a user may select at least one among information 172 representing whether the battery is in charge or not, information 173 regarding current battery capacity, information 174 regarding a battery charging method, and information 175 regarding an image mode, and display the selected information in the first picture 160. For example, referring to FIG. 17(a), the information 172 representing whether the battery is in charge or not, the information 173 regarding the current battery capacity, and the information 175 regarding the image mode may be selected. Thus, only the three types of information may be included in the first picture 160 as illustrated in FIG. 17(b).

Various embodiments of the ultrasonic probe 100 have been described above with reference to the drawings. However, the present disclosure is not limited thereto, and information regarding an operating state of an ultrasonic probe may include other information falling within a scope which may be employed by those of ordinary skill in the art, as well as the information illustrated in the drawings.

An ultrasonic diagnostic apparatus 300 in accordance with another embodiment of the present disclosure will be described below. Since the ultrasonic diagnostic apparatus 300 includes an ultrasonic probe 100 according to structural characteristics thereof, the characteristics of the ultrasonic probe 100 described above may also apply to the ultrasonic diagnostic apparatus 300. Thus, although the following description is focused on the characteristics of the ultrasonic diagnostic apparatus 300, it should not be understood that the characteristics of the ultrasonic probe 100 described above does not apply to the ultrasonic diagnostic apparatus 300. The characteristics of the ultrasonic probe 100 may also apply to the ultrasonic diagnostic apparatus 300.

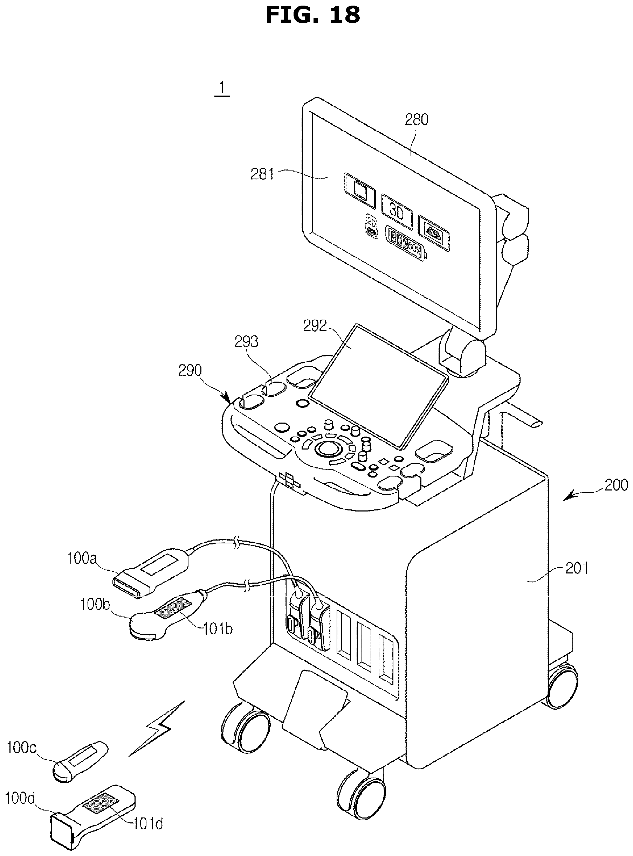

FIG. 18 illustrates the exterior of an ultrasonic diagnostic apparatus 300 in accordance with another embodiment of the present disclosure. The ultrasonic diagnostic apparatus 300 of FIG. 18 includes the same elements as the ultrasonic diagnostic apparatus 300 of FIG. 1 and will be described focusing on the differences from the ultrasonic diagnostic apparatus 300 below.

Referring to FIGS. 2 and 18, the ultrasonic diagnostic apparatus 300 may include at least one ultrasonic probe 100 having a main body 200 and a first display 150, a second display 280 coupled to the main body 200 and configured to output information received from the main body 200, and a second controller 220 for displaying a fourth picture 281 on the second display 280 when an operating state of the ultrasonic diagnostic apparatus 300 is switched to a standby state. The fourth picture 281 includes at least one among information regarding operating states of the ultrasonic probe 100 and the ultrasonic diagnostic apparatus 300. The information is set beforehand by a user.

In detail, the standby state means a state in which the ultrasonic diagnostic apparatus 300 is not in use by a user any longer and may correspond to the first time described above. For example, the first time may correspond to one minute, two minutes, three minutes, or the like. However, the first time is not limited thereto and may be variously changed according to an operating environment of the ultrasonic diagnostic apparatus 300. The first time may be directly set by a user.

Thus, when the ultrasonic diagnostic apparatus 300 does not receive an input from a user for the first time, the second controller 220 may cancel a picture currently displayed on the second display 280 and display the fourth picture 281 on the second display 280 as illustrated in FIG. 18 to prevent waste of power.

Here, the fourth picture 281 is a picture which includes at least one among information regarding operating states of the ultrasonic diagnostic apparatus 300 and the ultrasonic probe 100 and through which a user may view information regarding operations of the ultrasonic probe 100 or the ultrasonic diagnostic apparatus 300 without manipulating the ultrasonic probe 100 or the ultrasonic diagnostic apparatus 300. The fourth picture 281 is named differently from the second picture 180 described above to be differentiated from the second picture 180 but the second picture 180 and the fourth picture 281 have substantially the same characteristics. Thus, the fourth picture 281 is not described in detail here.

Furthermore, the ultrasonic diagnostic apparatus 300 may drive the ultrasonic diagnostic apparatus 300 using a battery (not shown) mounted therein when an external power source is cut off. In this case, for efficient use of the battery, the second controller 220 may display a fifth picture (not shown) by adjusting the types and brightness of information included in the fourth picture 281 on the basis of the capacity of the battery after the ultrasonic diagnostic apparatus 300 is switched to the standby state.

Furthermore, when any input is not received from a user for a third time after the ultrasonic diagnostic apparatus 300 is switched to the standby mode, the second controller 220 may power off the ultrasonic diagnostic apparatus 300 to prevent waste of power.

Here, the fifth picture has the same characteristics as the second picture 180 and the third time has the same characteristics as the third time described above. Thus, the fifth picture and the third time are not described in detail here.

Furthermore, the ultrasonic diagnostic apparatus 300 in accordance with an embodiment of the present disclosure may separately include a controller (not shown) dedicated to controlling the second display 280 as described above with reference to FIG. 2. In this case, when the ultrasonic diagnostic apparatus 300 enters the standby mode, the second display 280 may be controlled by turning off the second controller 220 which performs a relatively large number of operations and driving only the controller which performs a relatively small number of operations, thereby increasing power efficiency.

The controller controlling the second display 280 may be referred to variously as a controller of the second display 280, a standby screen controller, an AOD controller, or the like.

The ultrasonic diagnostic apparatus 300 in accordance with another embodiment may include a plurality of ultrasonic probes 100a, 100b, 100c, and 100d as illustrated in FIG. 18. An ultrasonic diagnostic apparatus according to the related art is inconvenient to use since a user has to manipulate all ultrasonic probes connected to the ultrasonic diagnostic apparatus so as to identify the ultrasonic probes.

In contrast, in the ultrasonic diagnostic apparatus 300 in accordance with another embodiment of the present disclosure, a user may be able to easily recognize ultrasonic probes connected to the ultrasonic diagnostic apparatus 300 through the fourth picture 281 output to the second display 280.

Such identification information may be provided to a user through the first display 150 provided on the ultrasonic probe 100, as well as through the fourth picture 281.

In detail, as illustrated in FIG. 18, the identification information may be provided to a user by displaying specific colors 101b and 101d on the first display 150. In this case, even if the user is located far from the ultrasonic probes 100b and 100d, the user may be able to intuitively recognize the ultrasonic probes 100b and 100d currently connected to the ultrasonic diagnostic apparatus 300 by viewing only the specific colors 101b and 101d. Alternatively, different colors may be displayed on displays of ultrasonic probes so that a user may easily differentiate the ultrasonic probes from one another.

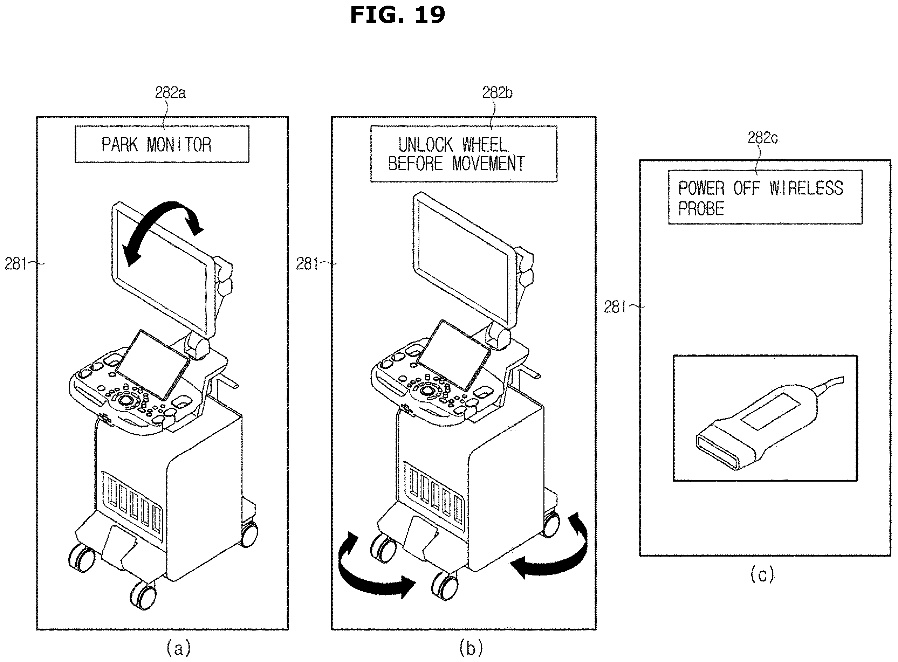

FIG. 19 illustrates various information as examples of the fourth picture 281 in accordance with another embodiment of the present disclosure.

That the ultrasonic diagnostic apparatus 300 is in the standby state means that it is highly probable that the ultrasonic diagnostic apparatus 300 is not in use by a user. Thus, in this case, the fourth picture 281 may include information regarding the ultrasonic diagnostic apparatus 300 that the user should notice, so that the user may easily manage the ultrasonic diagnostic apparatus 300.

In detail, information regarding monitor parking may be displayed as illustrated in FIG. 19(a) or information regarding wheels may be displayed as illustrated in FIG. 19(b). Alternatively, a picture including a message suggesting that an ultrasonic probe connected wirelessly be powered off may be displayed as illustrated in FIG. 19(c).

FIG. 19(b) displays information regarding locking of wheels among information regarding the wheels in accordance with an embodiment of the present disclosure but the present disclosure is not limited thereto and various current information regarding the wheels may be displayed. For example, information as to whether the wheels are in a fixed state in which they cannot be moved, in a linear fixed state in which they may be moved linearly, or a state in which they may be moved freely may be displayed so that a user may easily recognize information regarding the wheels.

Furthermore, the fourth picture 281 may include lately updated information regarding the ultrasonic diagnostic apparatus 300 received from an external server, medical information, etc. Alternatively, the fourth picture 281 may include an image captured by an ultrasonic probe, various information regarding a cable, etc.

When an image is displayed, a user may set colors and brightness of the image. When the information regarding the cable is displayed, information regarding a current connected state between the main body 200 and the cable or information regarding a degree to which the cable is wound may be displayed so that a user may easily arrange the cable.

Furthermore, although not shown, the fourth picture 281 may include information regarding a current state of the ultrasonic diagnostic apparatus 300. The information regarding the current state of the ultrasonic diagnostic apparatus 300 may include information regarding a current capacity of a battery of the ultrasonic diagnostic apparatus 300 or information regarding whether an external device such as a printer or the like is currently connected to the ultrasonic diagnostic apparatus 300. The fourth picture 281 may further include information regarding a contrast enhanced ultrasound (CEUS) image.

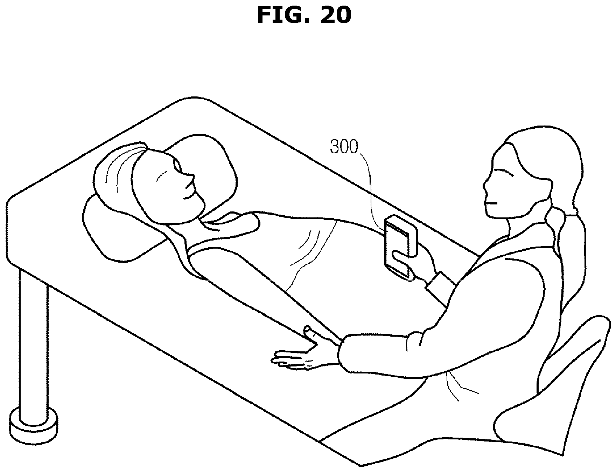

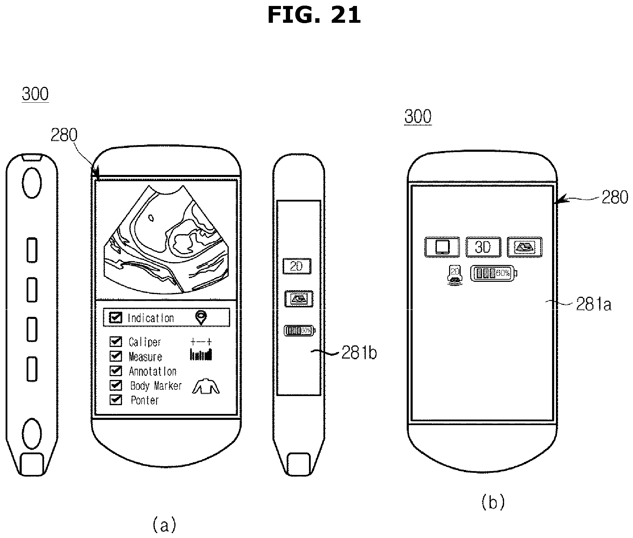

FIGS. 20 and 21 illustrate an ultrasonic diagnostic apparatus 300 in accordance with another embodiment of the present disclosure. FIG. 20 illustrates a case in which a user obtains an ultrasonic image using the ultrasonic diagnostic apparatus 300. FIG. 21 illustrates a picture output to a second display 280 of the ultrasonic diagnostic apparatus 300.

Although ultrasonic probes and ultrasonic diagnostic apparatuses have been described above separately from each other, the boundaries between ultrasonic probes and ultrasonic diagnostic apparatuses have been disappearing with advancement of technology. That is, a large number of small-sized ultrasonic diagnostic apparatuses capable of serving as an ultrasonic probe have been developed. Thus, a user may obtain an image of an object while viewing the image on a display using the ultrasonic diagnostic apparatus 300 having a small size as illustrated in FIG. 20.

Thus, when a user obtains an image using the ultrasonic diagnostic apparatus 300 having a small size, a fourth picture 281b may be output to another display provided on a side surface of the ultrasonic diagnostic apparatus 300 as illustrated in FIG. 21(a).

When the ultrasonic diagnostic apparatus 300 does not receive an input from a user for a predetermined time and is thus switched to the standby mode, a fourth picture 281a as described above may be output as illustrated in FIG. 21(b)
The features and effects of the present disclosure have been described above by describing various embodiments of the present disclosure.

In the case of an ultrasonic probe and an ultrasonic diagnostic apparatus according to the related art, a user should manipulate them several times to view information needed to control the ultrasonic probe and the ultrasonic diagnostic apparatus.

In contrast, according to the present disclosure, information set by a user may be viewed at any time through a display installed in an ultrasonic probe or an ultrasonic diagnostic apparatus. Thus, the user may easily obtain information regarding the ultrasonic probe or the ultrasonic diagnostic apparatus.

As is apparent from the above description, according to the present disclosure, information set by a user may be viewed through a display installed in an ultrasonic probe or an ultrasonic diagnostic apparatus at any time. Thus, the user may easily obtain and control information regarding the ultrasonic probe or the ultrasonic diagnostic apparatus.

While the present disclosure has been described with reference to embodiments and drawings, various changes and modification may be made from the above description by those of ordinary skill in the art. For example, an appropriate result may be obtained even when the technologies described above are performed in an order different from that described herein and/or even when elements such as systems, structures, devices, circuits described above are coupled or combined to each other in a method different from that described above or are replaced with equivalents thereto. Accordingly, it should be understood that other embodiments, examples, and equivalents to the claims fall within the scope of the claims described below.

Although a few embodiments of the present disclosure have been shown and described, it would be appreciated by those skilled in the art that changes may be made in these embodiments without departing from the principles of the disclosure, the scope of which is defined in the claims.

## Claims

1. An ultrasonic probe (100) comprising:
an ultrasonic transceiver (110) configured to transmit an ultrasonic signal to an object and receive a signal reflected from the object (99);
a first display (150) configured to receive information from a user or output information received from an ultrasonic diagnostic apparatus (300); and
a first controller (140) configured to display a first picture on the first display (150) when an operating state of the ultrasonic probe (100) is switched to a standby state, wherein the first picture includes information regarding the operating state of the ultrasonic probe (100), and **characterized in that**
the type of information regarding the operating state of the ultrasonic probe included in the first picture is configured to be set by the user, wherein
the first display (150) comprises an OLED (Organic Light-Emitting Device) panel (184),
wherein the first controller (140) is configured to control the first display to drive only pixels corresponding to the area in which the information regarding the operation state of the ultrasonic probe (100) is displayed in the first picture.

2. The ultrasonic probe according to claim 1, wherein the first controller (140) is configured to switch the operating state of the ultrasonic probe (100) to the standby state when any input is not received from the user for a first time, when the user presses a lock button of the ultrasonic probe (100), or when the ultrasonic probe (100) is mounted in a holder (292) of the ultrasonic diagnostic apparatus.

3. The ultrasonic probe according to claim 1, wherein the first controller (140) is configured to display a second picture on the first display (150) when any input is not received from the user for a second time after the operating state of the ultrasonic probe (100) is switched to the standby state, wherein the second picture is darker than the first picture and includes a smaller number of types of information than a number of types of the information included in the first picture.

4. The ultrasonic probe according to claim 1, wherein the first controller (140) is configured to control at least one among brightness of the first picture and types of colors to be expressed in the first picture on the basis of a capacity of a battery of the ultrasonic probe (100).

5. The ultrasonic probe according to claim 1, wherein the first controller (140) is configured to display the information regarding the operating state of the ultrasonic probe (100) in the form of an icon.

6. The ultrasonic probe according to claim 5, wherein the first display (150) comprises a touch screen panel, and
when the user touches the icon, the first controller (140) is configured to display information related to the icon or executes a program related to the icon.

7. The ultrasonic probe according to claim 3, wherein the first controller (140) is configured to power off the ultrasonic probe (100) when the ultrasonic probe (100) does not receive any input from the user for a third time.

8. The ultrasonic probe according to claim 3, wherein the first controller (140) is configured to switch the ultrasonic probe (100) to the operating state preceding the standby mode when the ultrasonic probe (100) receives an input from the user within a third time, when the user cancels pressing a lock button of the ultrasonic probe (100), or when the ultrasonic probe (100) is separated from a holder (292) of the ultrasonic diagnostic apparatus (300).

9. The ultrasonic probe according to claim 1, wherein the information regarding the operating state comprises information identifying the ultrasonic diagnostic apparatus (400, 500, 600) paired with the ultrasonic probe (100).

10. The ultrasonic probe according to claim 1, wherein the information regarding the operating state comprises at least one of:
a state of a battery of the ultrasonic probe (100);
information as to whether the battery is in charging or not; and
a charging method.

11. The ultrasonic probe according to claim 1, wherein the information regarding the operating state comprises information identifying a mode in which an image captured by the ultrasonic probe (100) is output.

12. The ultrasonic probe according to claim 1, wherein the information regarding the operating state comprises information identifying a program used by the ultrasonic probe (100).

## Patentansprüche

1. Ultraschallsonde (100), die Folgendes umfasst:
eine Ultraschall-Sende- und Empfangsvorrichtung (110), die dazu ausgelegt ist, ein Ultraschallsignal an ein Objekt zu senden und ein von dem Objekt (99) reflektiertes Signal zu empfangen;
eine erste Anzeige (150), die dazu ausgelegt ist, von einem Benutzer stammende Informationen oder empfangene, von einer Ultraschalldiagnosevorrichtung (300) stammende Informationen zu empfangen; und
eine erste Steuerung (140), die dazu ausgelegt ist, ein erstes Bild auf der ersten Anzeige (150) anzuzeigen, wenn ein Betriebszustand der Ultraschallsonde (100) in einen Standby-Zustand umgeschaltet wird, wobei das erste Bild Informationen über den Betriebszustand der Ultraschallsonde (100) enthält, und **dadurch gekennzeichnet, dass**
die Art von in dem ersten Bild enthaltenen Informationen über den Betriebszustand der Ultraschallsonde dazu ausgelegt ist, von dem Benutzer eingestellt zu werden,
wobei die erste Anzeige (150) eine OLED-Tafel (Organic Light-Emitting Device, Organische Leuchtdiode) (184) umfasst,
wobei die erste Steuerung (140) dazu ausgelegt ist, die erste Anzeige dazu zu steuern, nur Pixel anzuregen, die dem Bereich entsprechen, in dem die Informationen über den Betriebszustand der Ultraschallsonde (100) in dem ersten Bild angezeigt werden.

2. Ultraschallsonde nach Anspruch 1, wobei die erste Steuerung (140) dazu ausgelegt ist, den Betriebszustand der Ultraschallsonde (100) in den Standby-Zustand umzuschalten, wenn während eines ersten Zeitraums keine von dem Benutzer stammende Eingabe empfangen wird, wenn der Benutzer eine Verriegelungstaste der Ultraschallsonde (100) drückt, oder wenn die Ultraschallsonde (100) in einer Halterung (292) der Ultraschalldiagnosevorrichtung gelagert wird.

3. Ultraschallsonde nach Anspruch 1, wobei die erste Steuerung (140) dazu ausgelegt ist, ein zweites Bild auf der ersten Anzeige (150) anzuzeigen, wenn während eines zweiten Zeitraums, nachdem der Betriebszustand der Ultraschallsonde (100) in den Standby-Zustand umgeschaltet wurde, keine von dem Benutzer stammende Eingabe empfangen wird, wobei das zweite Bild dunkler als das erste Bild ist und eine geringere Anzahl von Arten von Informationen enthält als die Anzahl von in dem ersten Bild enthaltenen Arten von Informationen.

4. Ultraschallsonde nach Anspruch 1, wobei die erste Steuerung (140) dazu ausgelegt ist, die Helligkeit des ersten Bildes und/oder die Arten von Farben, die in dem ersten Bild dargestellt werden sollen, basierend auf der Kapazität einer Batterie in der Ultraschallsonde (100) zu steuern.

5. Ultraschallsonde nach Anspruch 1, wobei die erste Steuerung (140) dazu ausgelegt ist, die Informationen über den Betriebszustand der Ultraschallsonde (100) in der Form eines Symbols anzuzeigen.

6. Ultraschallsonde nach Anspruch 5, wobei die erste Anzeige (150) eine Berührungsbildschirmtafel umfasst, und
wenn der Benutzer das Symbol berührt, die erste Steuerung (140) dazu ausgelegt ist, Informationen in Verbindung mit dem Symbol anzuzeigen oder ein Programm in Verbindung mit dem Symbol durchführt.

7. Ultraschallsonde nach Anspruch 3, wobei die erste Steuerung (140) dazu ausgelegt ist, die Ultraschallsonde (100) auszuschalten, wenn die Ultraschallsonde (100) während eines dritten Zeitraums keine von dem Benutzer stammende Eingabe empfängt.

8. Ultraschallsonde nach Anspruch 3, wobei die erste Steuerung (140) dazu ausgelegt ist, den Betriebszustand der Ultraschallsonde (100) in einen dem Standby-Zustand vorausgehenden Betriebszustand umzuschalten, wenn die Ultraschallsonde (100) während eines dritten Zeitraums eine von dem Benutzer stammende Eingabe empfängt, wenn der Benutzer das Drücken der Verriegelungstaste der Ultraschallsonde (100) aufhebt, oder wenn die Ultraschallsonde (100) von einer Halterung (292) der Ultraschalldiagnosevorrichtung (300) getrennt wird.

9. Ultraschallsonde nach Anspruch 1, wobei die Informationen über den Betriebszustand Informationen zur Identifizierung der mit der Ultraschallsonde (100) gepaarten Ultraschalldiagnosevorrichtung (400, 500, 600) umfassen.

10. Ultraschallsonde nach Anspruch 1, wobei die Informationen über den Betriebszustand wenigstens eine der Folgenden umfassen:
einen Zustand einer Batterie der Ultraschallsonde (100);
Informationen darüber, ob die Batterie gerade aufgeladen wird oder nicht; und
ein Aufladeverfahren.

11. Ultraschallsonde nach Anspruch 1, wobei die Informationen über den Betriebszustand Informationen zur Identifizierung eines Modus, in dem ein von der Ultraschallsonde (100) aufgenommenes Bild ausgegeben wird, umfassen.

12. Ultraschallsonde nach Anspruch 1, wobei die Informationen über den Betriebszustand Informationen zur Identifizierung eines von der Ultraschallsonde (100) verwendeten Programms umfassen.

## Revendications

1. Sonde ultrasonore (100) comprenant :
un émetteur-récepteur ultrasonore (110) conçu pour émettre un signal ultrasonore à destination d'un objet et pour recevoir un signal réfléchi par l'objet (99),
un premier dispositif d'affichage (150) conçu pour recevoir des informations provenant d'un utilisateur ou pour transmettre des informations reçues en provenance d'un appareil de diagnostic échographique (300), et
un premier organe de commande (140) conçu pour afficher une première image sur le premier dispositif d'affichage (150) lorsque l'état d'exécution de la sonde ultrasonore (100) passe à un état de veille, ladite première image comportant des informations concernant l'état d'exécution de la sonde ultrasonore (100) ; et **caractérisée en ce que**
le type d'information concernant l'état d'exécution de la sonde ultrasonore présent sur la première image est conçu pour être défini par l'utilisateur ;
ledit premier dispositif d'affichage (150) comprenant un écran OLED (diodes électroluminescentes organiques) (184),
ledit premier organe de commande (140) étant conçu pour commander le premier dispositif d'affichage afin d'exciter uniquement les pixels correspondant à la zone dans laquelle les informations concernant l'état d'exécution de la sonde ultrasonore (100) sont affichées sur la première image.

2. Sonde ultrasonore selon la revendication 1, dans laquelle le premier organe de commande (140) est conçu pour faire passer l'état d'exécution de la sonde ultrasonore (100) à l'état de veille lorsqu'aucune entrée n'est reçue de la part de l'utilisateur pendant une première durée, lorsque l'utilisateur appuie sur un bouton de verrouillage de la sonde ultrasonore (100), ou lorsque la sonde ultrasonore (100) est montée dans un logement (292) de l'appareil de diagnostic échographique.

3. Sonde ultrasonore selon la revendication 1, dans laquelle le premier organe de commande (140) est conçu pour afficher une deuxième image sur le premier dispositif d'affichage (150) lorsqu'aucune entrée n'est reçue de la part de l'utilisateur pendant une deuxième durée après que l'état d'exécution de la sonde ultrasonore (100) est passé à l'état de veille, ladite deuxième image étant plus foncée que la première image et comportant un moindre nombre de types d'information que le nombre de types d'information présents sur la première image.

4. Sonde ultrasonore selon la revendication 1, dans laquelle le premier organe de commande (140) est conçu pour commander la luminosité de la première image et/ou les types de couleurs à exprimer sur la première image en fonction de la capacité d'une batterie de la sonde ultrasonore (100).

5. Sonde ultrasonore selon la revendication 1, dans laquelle le premier organe de commande (140) est conçu pour afficher les informations concernant l'état d'exécution de la sonde ultrasonore (100) sous la forme d'une icône.

6. Sonde ultrasonore selon la revendication 5, dans laquelle le premier dispositif d'affichage (150) comprend un écran tactile, et
lorsque l'utilisateur touche l'icône, le premier organe de commande (140) est conçu pour afficher des informations en lien avec l'icône ou exécute un programme en lien avec l'icône.

7. Sonde ultrasonore selon la revendication 3, dans laquelle le premier organe de commande (140) est conçu pour mettre hors tension la sonde ultrasonore (100) lorsque la sonde ultrasonore (100) ne reçoit aucune entrée de la part de l'utilisateur pendant une troisième durée.

8. Sonde ultrasonore selon la revendication 3, dans laquelle le premier organe de commande (140) est conçu pour faire passer la sonde ultrasonore (100) à l'état d'exécution précédant le mode de veille lorsque la sonde ultrasonore (100) reçoit une entrée de l'utilisateur en l'espace d'une troisième durée, lorsque l'utilisateur annule l'action d'un bouton de verrouillage de la sonde ultrasonore (100), ou lorsque la sonde ultrasonore (100) est séparée d'un logement (292) présent sur appareil de diagnostic échographique (300).

9. Sonde ultrasonore selon la revendication 1, dans laquelle les informations concernant l'état d'exécution comprennent des informations identifiant l'appareil de diagnostic échographique (400, 500, 600) associé à la sonde ultrasonore (100).

10. Sonde ultrasonore selon la revendication 1, dans laquelle les informations concernant l'état d'exécution comprennent :
le niveau de la batterie de la sonde ultrasonore (100),
des informations relatives au fait que la batterie est en chargement ou non, et/ou
la méthode de chargement.

11. Sonde ultrasonore selon la revendication 1, dans laquelle les informations concernant l'état d'exécution comprennent des informations identifiant le mode selon lequel une image prise par la sonde ultrasonore (100) est transmise.

12. Sonde ultrasonore selon la revendication 1, dans laquelle les informations concernant l'état d'exécution comprennent des informations identifiant un programme utilisé par la sonde ultrasonore (100).
